(19)

Europäisches
Patentamt
European
Patent Office
Office européen
des brevets

(11) **EP 3 896 999 A1**

(12) **EUROPEAN PATENT APPLICATION**

(43) Date of publication:
**20.10.2021 Bulletin 2021/42**

(51) Int Cl.:
**H04R 25/00** (2006.01)     **G10L 21/0364** (2013.01)

(21) Application number: **20197642.0**

(22) Date of filing: **23.09.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.04.2020 US 202016851048
13.08.2020 US 202016992407**

(71) Applicant: **Mimi Hearing Technologies GmbH
10245 Berlin (DE)**

(72) Inventors:
- **GUZ, Marina
  10245 Berlin (DE)**
- **KLIMCZAK, Ryan
  10245 Berlin (DE)**

(74) Representative: **MERH-IP Matias Erny Reichl
Hoffmann
Patentanwälte PartG mbB
Paul-Heyse-Strasse 29
80336 München (DE)**

(54) **SYSTEMS AND METHODS FOR A HEARING ASSISTIVE DEVICE**

(57) Disclosed are systems and methods for ambient sound enhancement on a mobile device. A user hearing profile is generated and a set of ambient sound enhancement digital signal processing (DSP) parameters is calculated for a sound enhancement algorithm, based at least in part on the user hearing profile. In response to a user initiating an ambient sound enhancement function on a mobile computing device, at least one set of calculated ambient sound enhancement DSP parameters is retrieved. Ambient sound is captured with a microphone of the mobile computing device and processed with an ambient sound enhancement DSP. The ambient sound enhancement DSP is parameterized with the retrieved set of calculated ambient sound enhancement DSP parameters and the DSP enhanced processed audio signal is outputted to a transducer of the mobile device.

1001 OBTAIN USER'S MASKING COUNTOUR CURVE

1002 DETERMINE TARGET MASKING CONTOUR CURVE

1003 COMPARE USER'S MASKING CONTOUR CURVE WITH TARGET MASKING CONTOUR CURVE

1004 OUTPUT CALCULATED PARAMETERS

FIG. 10

EP 3 896 999 A1

**Description**

**CROSS-REFERENCE TO RELATED APPLICATIONS**

This application claims priority to US 16/851,048 entitled "SYSTEMS AND METHODS FOR PROVIDING CONTENT-SPECIFIC, PERSONALIZED AUDIO REPLAY ON CUSTOMER DEVICES," filed April 16, 2020, which is incorporated by reference herein in its entirety.

**FIELD OF INVENTION**

This invention relates generally to the field of digital signal processing, audio engineering and audiology - more specifically systems and methods for a hearing assistive device, for example having a user's hearing test parameterize a sound enhancement algorithm that can process ambient sound through a mobile device.

**BACKGROUND**

Hearing aids, although effective for improving speech comprehension for listeners, are still extremely expensive and inaccessible for the vast majority of hearing impaired (HI) individuals. Furthermore, the use of hearing aids has been subject to social stigmatization, despite the prevalence of hearing loss across all age groups. Cheaper hearing assistive devices, such as over the counter sound enhancement ear buds, provide a solution to this problem - but fall short due to limitations in processing capacity, as well as inadequate testing methodologies and ineffective signal processing techniques.

The most common technique employed by hearing assistive devices consists of a simple increase in wide spectrum gain (i.e. volume enhancement). Less commonly, simple equalization (EQ) handset applications have been utilized. These applications apply gain(s) to frequencies in which a listener exhibits raised thresholds (as determined through an audiogram). Both techniques may enable a listener to better perceive conversation, however, the listener may simultaneously or subsequently experience loudness discomfort. This is because listeners with sensorineural hearing loss have similar, or even reduced, discomfort thresholds when compared to normal hearing listeners, despite the hearing thresholds of such HI listeners being raised relative to normal hearing listeners. To this extent, the dynamic range of HI listeners is narrower and simply adding EQ or wide spectrum gain would be detrimental to the long-term hearing health of these HI listeners.

Although hearing loss typically begins at higher frequencies, listeners who are aware that they have hearing loss do not typically complain about the absence of high frequency sounds. Instead, they report difficulties listening in a noisy environment and in hearing out the details in a complex mixture of sounds, such as in a normal conversation at restaurant or coffeeshop. In essence, off frequency sounds more readily mask information with energy in other frequencies for HI individuals - conversations that were once clear become muddled by background noise, e.g. background noises mask the sound-of-interest. As hearing deteriorates, the signal-conditioning capabilities of the ear begin to break down, and thus HI listeners need to expend more mental effort to make sense of sounds of interest in complex acoustic scenes (or miss the information entirely). A raised threshold in an audiogram is not merely a reduction in aural sensitivity, but a result of the malfunction of some deeper processes within the auditory system that have implications beyond the detection of faint sounds. To this extent, the use of suprathreshold data, such as masked threshold (MT) data, to parameterize a sound enhancement DSP for a hearing assistive device would better measure the increase masking that occurs with hearing deterioration.

As the majority of individuals have access to a smartphone (with high processing capabilities) and a set of headphones and/or ear pods (by one estimate, 45% of the world), this presents a global opportunity to provide greater accessibility to hearing technology with improved hearing test methodologies that will help HI individuals.

Accordingly, it is an aspect of the present disclosure to provide systems and methods for a hearing assistive device, for example having a user's hearing test parameterize a sound enhancement algorithm that can process ambient sounds through a mobile device.

**SUMMARY**

According to an aspect of the present disclosure, a method for ambient sound enhancement on a mobile device comprises: generating a user hearing profile; calculating at least one set of ambient sound enhancement digital signal processing (DSP) parameters for each of one or more sound enhancement algorithms, the calculation of the ambient sound enhancement DSP parameters based at least in part on the user hearing profile; in response to a user initiating an ambient sound enhancement function on a mobile computing device, retrieving the at least one set of calculated ambient sound enhancement DSP parameters; capturing ambient sound with at least one microphone of the mobile computing device; processing the captured ambient sound with an ambient sound enhancement DSP to generate a DSP enhanced processed audio signal, wherein the ambient sound enhancement DSP is parameterized with the retrieved set of calculated ambient sound enhancement DSP parameters; and outputting the DSP enhanced processed audio signal to a transducer of the mobile device.

In a further aspect of the present disclosure, capturing the ambient sound is performed in substantially real time with one or more of: processing the captured ambient sound with the ambient sound enhancement DSP to generate the DSP enhanced processed audio signal; and outputting the DSP enhanced processed audio signal to the transducer of the mobile device.

In a further aspect of the present disclosure, the retrieved set of calculated ambient sound enhancement DSP parameters corresponds to a sound enhancement algorithm associated with the user's mobile computing device or indicated by a user input to the mobile computing device.

In a further aspect of the present disclosure, the sound enhancement algorithm associated with the user's mobile computing device is selected from a plurality of available sound enhancement algorithms configured in a local storage of the user's mobile computing device.

In a further aspect of the present disclosure, the selection of the sound enhancement algorithm from the plurality of sound enhancement algorithms is based at least in part on an analysis of the ambient sound captured by the user's mobile computing device.

In a further aspect of the present disclosure, the method further comprises one or more of processing the captured ambient sound to: attenuate sound not originating in front of the user or the microphone of the user's mobile computing device that captured the ambient sound, by applying a directional processing algorithm to the captured ambient sound or the DSP enhanced processed audio signal; and attenuate sounds that have typical characteristics of noise, regardless of the direction of arrival, by applying one or more digital noise reduction algorithms.

In a further aspect of the present disclosure, the user hearing profile is generated by conducted at least one hearing test on a mobile computing device.

In a further aspect of the present disclosure, the mobile computing device is the mobile computing device associated with the user.

In a further aspect of the present disclosure, the hearing test is one or more of a masked threshold test (MT test), a pure tone threshold test (PTT test), a psychophysical tuning curve test (PTC test), or a cross frequency simultaneous masking test (xF-SM test).

In a further aspect of the present disclosure, the user hearing profile is generated at least in part by analyzing a user input of demographic information to thereby interpolate a representative hearing profile.

In a further aspect of the present disclosure, the user input of demographic information includes an age of the user.

In a further aspect of the present disclosure, the sound enhancement algorithm is a multiband dynamic processor; and the at least one set of calculated ambient sound enhancement DSP parameters includes one or more ratio values and gain values.

In a further aspect of the present disclosure, the at least one set of calculated ambient sound enhancement DSP parameters is stored on a remote server; and retrieving the at least one set of calculated ambient sound enhancement DSP parameters comprises receiving a requested set of calculated ambient sound enhancement DSP parameters at the mobile computing device from the remote server.

In a further aspect of the present disclosure, the at least one set of calculated ambient sound enhancement DSP parameters is stored locally on the mobile computing device; and retrieving the at least one set of calculated ambient sound enhancement DSP parameters comprises accessing a local storage of the mobile computing device.

In a further aspect of the present disclosure, calculating the at least one set of ambient sound enhancement DSP parameters is performed on a remote server.

In a further aspect of the present disclosure, calculating the at least one set of ambient sound enhancement DSP parameters is performed by a processor of the user's mobile computing device.

In a further aspect of the present disclosure, the hearing test measures masking threshold curves within a range of frequencies from 250 Hz to 12 kHz.

In a further aspect of the present disclosure, the at least one set of calculated ambient sound enhancement DSP parameters is determined via one or more of: a best fit of the user hearing profile with previously inputted hearing data within a database; or a fitted mathematical function derived from plotted hearing and DSP parameter data.

In a further aspect of the present disclosure, the parameters associated with the best fit of the user hearing profile and the previously inputted hearing data are selected to correspond to a user's parameters.

In a further aspect of the present disclosure, the best fit is determined by one or more of average Euclidean distance and root mean square difference.

Unless otherwise defined, all technical terms used herein have the same meaning as commonly understood by one of ordinary skill in the art to which this technology belongs.

The term "sound enhancement algorithm", as used herein, is defined as any digital signal processing (DSP) algorithm that processes an audio signal to enhance the clarity of the signal to a listener. The DSP algorithm may be, for example: an equalizer, an audio processing function that works on the subband level of an audio signal, a multiband compressive system, or a non-linear audio processing algorithm.

The term "hearing test", as used herein, is any test that evaluates a user's hearing health, more specifically a hearing test administered using any transducer that outputs a sound wave. The test may be a threshold test or a suprathreshold test, including, but not limited to, a psychophysical tuning curve (PTC) test, a masked threshold (MT) test, a temporal fine structure test (TFS), temporal masking curve test and a speech in noise test.

The term "server", as used herein, generally refers to a computer program or device that provides functionalities for other programs or devices.

The term "headphone" or "earphone", as used herein, is any earpiece bearing a transducer that outputs soundwaves into the ear. The earphone may be a wireless hearable, a corded or wireless headphone, a hearable device, or any pair of earbuds.

The above aspects disclosed for the proposed method may be applied in a similar way to an apparatus or system having at least one processor and at least one memory to store programming instructions or computer program code and data, the at least one memory and the computer program code configured to, with the at least one processor, cause the apparatus at least to perform the above functions. Alternatively, the above apparatus may be implemented by circuitry.

According to another broad aspect, a computer program comprising instructions for causing an apparatus to perform any of the above methods is disclosed. Furthermore, a computer readable medium comprising program instructions for causing an apparatus to perform any of the above methods is disclosed.

Furthermore, a non-transitory computer readable medium is disclosed, comprising program instructions stored thereon for performing the above functions.

Implementations of the disclosed apparatus may include using, but not limited to, one or more processor, one or more application specific integrated circuit (ASIC) and/or one or more field programmable gate array (FPGA). Implementations of the apparatus may also include using other conventional and/or customized hardware such as software programmable processors.

It will be appreciated that method steps and apparatus features may be interchanged in many ways. In particular, the details of the disclosed apparatus can be implemented as a method, as the skilled person will appreciate.

Other and further embodiments of the present disclosure will become apparent during the course of the following discussion and by reference to the accompanying drawings.

## BRIEF DESCRIPTION OF THE DRAWINGS

In order to describe the manner in which the above-recited and other advantages and features of the disclosure can be obtained, a more particular description of the principles briefly described above will be rendered by reference to specific embodiments thereof, which are illustrated in the appended drawings. Understand that these drawings depict only example embodiments of the disclosure and are not therefore to be considered to be limiting of its scope, the principles herein are described and explained with additional specificity and detail through the use of the accompanying drawings in which:

FIG. 1 illustrates example graphs showing deterioration of human audiograms with age;
FIG. 2 illustrates example graphs showing deterioration of masking thresholds with age;
FIG. 3 illustrates an exemplary method of a hearing assistive device;
FIG. 4 illustrates an example of PTC and MT test paradigms;
FIG. 5 illustrates an example of a cross frequency simultaneous masking (xF-SM) paradigm for an MT test;
FIG. 6 illustrates an example embodiment of a hearing assistive device;
FIG. 7 illustrates an example embodiment of a hearing assistive device;
FIG. 8 illustrates an exemplary multiband dynamic processing system;
FIG. 9 illustrates a method for attaining DSP parameters from user hearing data through the optimization of perceptually relevant information;
FIG. 10 illustrates a method of attaining ratio and threshold parameters from a user masking contour curve;
FIG. 11 illustrates a graph for attaining ratio and threshold parameters from a user PTC curve;
FIGS. 12A-C conceptually illustrate masked threshold curve widths for three different users, which can be used for best fit and/or nearest fit calculations;
FIG. 13 conceptually illustrates audiogram plots for three different users x, y and z, data points which can be used for best fit and / or nearest fit calculations;
FIG. 14 illustrates-a method for parameter calculation using a best-fit approach;

FIG. 15 illustrates a method for parameter calculation using an interpolation of nearest-fitting hearing data;

FIG. 16 illustrates an example system embodiment.

## DETAILED DESCRIPTION

Various embodiments of the disclosure are discussed in detail below. While specific implementations are discussed, it should be understood that this is done for illustration purposes only. A person skilled in the relevant art will recognize that other components and configurations may be used without parting from the spirit and scope of the disclosure. Thus, the following description and drawings are illustrative and are not to be construed as limiting the scope of the embodiments described herein. Numerous specific details are described to provide a thorough understanding of the disclosure. However, in certain instances, well-known or conventional details are not described in order to avoid obscuring the description. References to one or an embodiment in the present disclosure can be references to the same embodiment or any embodiment; and, such references mean at least one of the embodiments.

Reference to "one embodiment" or "an embodiment" means that a particular feature, structure, or characteristic described in connection with the embodiment is included in at least one embodiment of the disclosure. The appearances of the phrase "in one embodiment" in various places in the specification are not necessarily all referring to the same embodiment, nor are separate or alternative embodiments mutually exclusive of other embodiments. Moreover, various features are described which may be exhibited by some embodiments and not by others.

The terms used in this specification generally have their ordinary meanings in the art, within the context of the disclosure, and in the specific context where each term is used. Alternative language and synonyms may be used for any one or more of the terms discussed herein, and no special significance should be placed upon whether or not a term is elaborated or discussed herein. In some cases, synonyms for certain terms are provided. A recital of one or more synonyms does not exclude the use of other synonyms. The use of examples anywhere in this specification including examples of any terms discussed herein is illustrative only and is not intended to further limit the scope and meaning of the disclosure or of any example term. Likewise, the disclosure is not limited to various embodiments given in this specification.

Without intent to limit the scope of the disclosure, examples of instruments, apparatus, methods and their related results according to the embodiments of the present disclosure are given below. Note that titles or subtitles may be used in the examples for convenience of a reader, which in no way should limit the scope of the disclosure. Unless otherwise defined, technical and scientific terms used herein have the meaning as commonly understood by one of ordinary skill in the art to which this disclosure pertains. In the case of conflict, the present document, including definitions will control.

Additional features and advantages of the disclosure will be set forth in the description which follows, and in part will be obvious from the description, or can be learned by practice of the herein disclosed principles. The features and advantages of the disclosure can be realized and obtained by means of the instruments and combinations particularly pointed out in the appended claims. These and other features of the disclosure will become more fully apparent from the following description and appended claims or can be learned by the practice of the principles set forth herein.

Various example embodiments of the disclosure are discussed in detail below. While specific implementations are discussed, it should be understood that this is done for illustration purposes only. A person skilled in the relevant art will recognize that other components and configurations may be used without departing from the spirit and scope of the present disclosure.

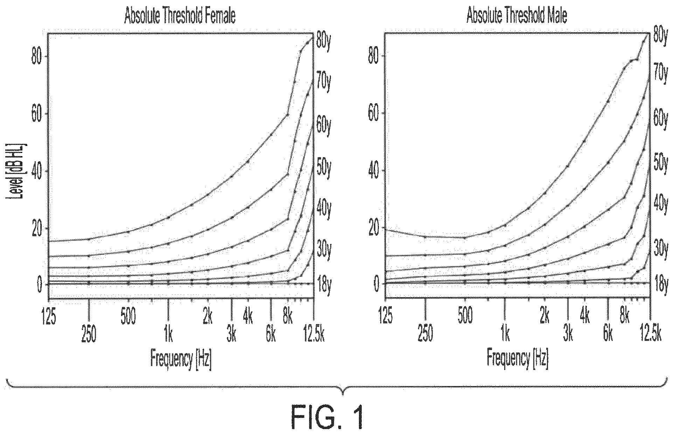

It is an aspect of the present disclosure to provide systems and methods for a hearing assistive device. FIGS. 1-2 underscore the importance of sound enhancement, illustrating the deterioration of a listener's hearing ability over time. Past the age of 20 years old, humans begin to lose their ability to hear higher frequencies, as illustrated by FIG. 1 (albeit above the spectrum of human voice). This steadily becomes worse with age as noticeable declines within the speech frequency spectrum are apparent around the age of 50 or 60. However, these pure tone audiometry findings mask a more complex problem as the human ability to understand speech may decline much earlier. Although hearing loss typically begins at higher frequencies, listeners who are aware that they have hearing loss do not typically complain about the absence of high frequency sounds. Instead, they report difficulties listening in a noisy environment and in hearing out the details in a complex mixture of sounds, such as in a telephone call. In essence, off-frequency sounds more readily mask a frequency of interest for hearing impaired individuals - conversation that was once clear and rich in detail becomes muddled. As hearing deteriorates, the signal-conditioning capabilities of the ear begin to break down, and thus hearing-impaired listeners need to expend more mental effort to make sense of sounds-of interest in complex acoustic scenes (or miss the information entirely). A raised threshold in an audiogram is not merely a reduction in aural sensitivity, but a result of the malfunction of some deeper processes within the auditory system that have implications beyond the detection of faint sounds.

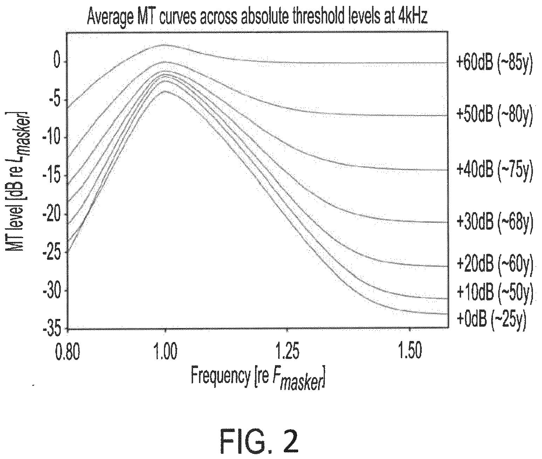

To this extent, FIG. 2 illustrates key, discernable age trends in suprathreshold hearing. Through the collection of large datasets, key age trends can be ascertained, allowing for the accurate parameterization of enhancement DSP algorithms. In a multiband compressive system, for example, the threshold and ratio values of each sub-band signal dynamic range compressor (DRC) can be modified to reduce problematic areas of frequency masking, while post-compression sub-band signal gain can be further applied in the relevant areas. Masked threshold curves depicted in FIG. 2 represent a similar paradigm for measuring masked threshold. A narrow band of noise, in this instance around 4kHz, is fixed while a probe tone sweeps from 50% of the noise band center frequency to 150% of the noise band center frequency. Again, key age trends can be ascertained from the collection of large MT datasets.

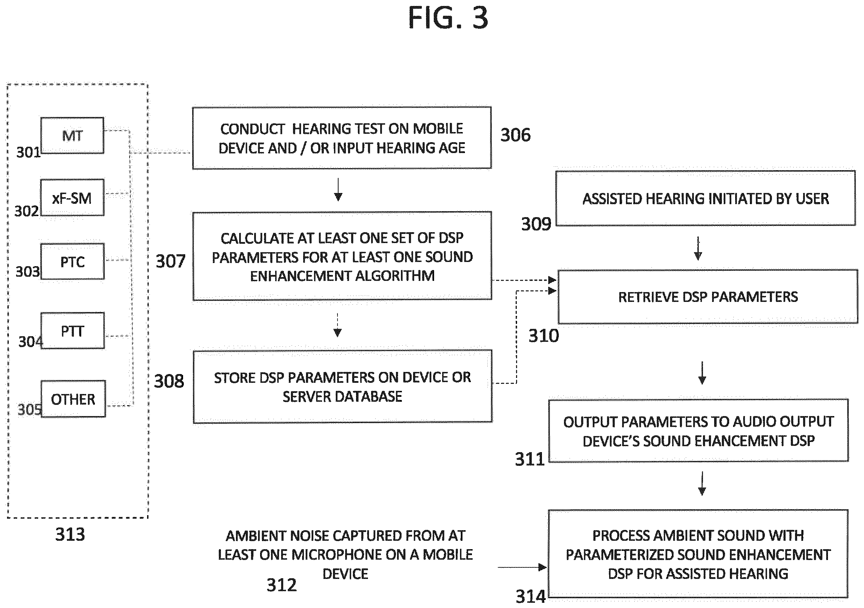

FIG. 3 illustrates an exemplary embodiment according to aspects of the present disclosure, in which a user's hearing test and/or hearing profile parameterizes a sound enhancement algorithm that can process ambient sounds through a user's mobile device. First, a hearing test is conducted 306 on a mobile device. Alternatively, a user may just input their age, which would then be used to generate and input a representative hearing profile based on their age. The hearing test may be provided by one or more hearing test options, including but not limited to: a masked threshold test (MT test) 301, a cross frequency simultaneous masking test (xF-SM) 302, a psychophysical tuning curve test (PTC test) 303, a pure tone threshold test (PTT test) 304, or various other suprathreshold tests 305. Next, hearing test results are used to calculate 307 at least one set of DSP parameters for at least one sound enhancement algorithm, which are then stored on the device or on a remote server database 308. When a user initiates assisted hearing 309 on their mobile device, DSP parameters are retrieved 310 and outputted 311 to the mobile device's sound enhancement DSP. Subsequently, when ambient sound is captured from one or more microphones on the mobile device 312, the ambient sound is processed 314 with the parameterized sound enhancement DSP for assisted hearing specific to the specific user's hearing test or hearing test results.

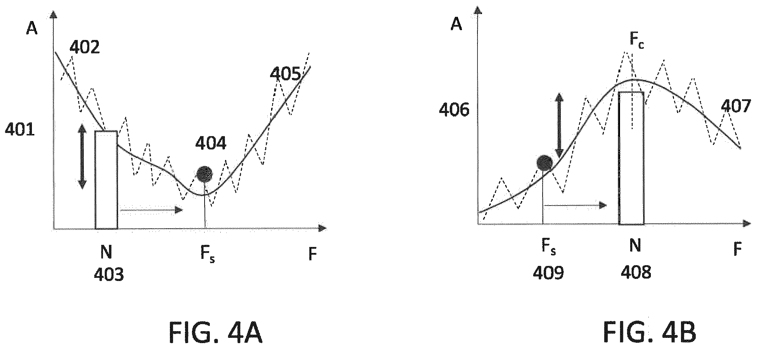

FIGS. 4A-B illustrate a method in which a PTC test 401 (FIG. 4A) or MT test 406 (FIG. 4B) may be conducted to assess a user's hearing. A psychophysical tuning curve (PTC), consisting of a frequency selectivity contour 404 extracted via behavioral testing, provides useful data to determine an individual's masking contours. In one embodiment of the test seen in FIG. 4A, a masking band of noise 403 is gradually swept across frequency, from below the probe frequency 404 to above the probe frequency 404. The user then responds when they can hear the probe and stops responding when they no longer hear the probe. This gives a jagged trace 405 that can then be interpolated to estimate the underlying characteristics of the auditory filter. Other methodologies may also be employed to attain user masking contour curves without departing from the scope of the present disclosure. For instance, an inverse paradigm may be used in which a probe tone 409 is swept across frequency while a masking band of noise 408 is fixed at a center frequency (known as a "masked threshold test" or "MT test", as depicted in FIG. 4B).

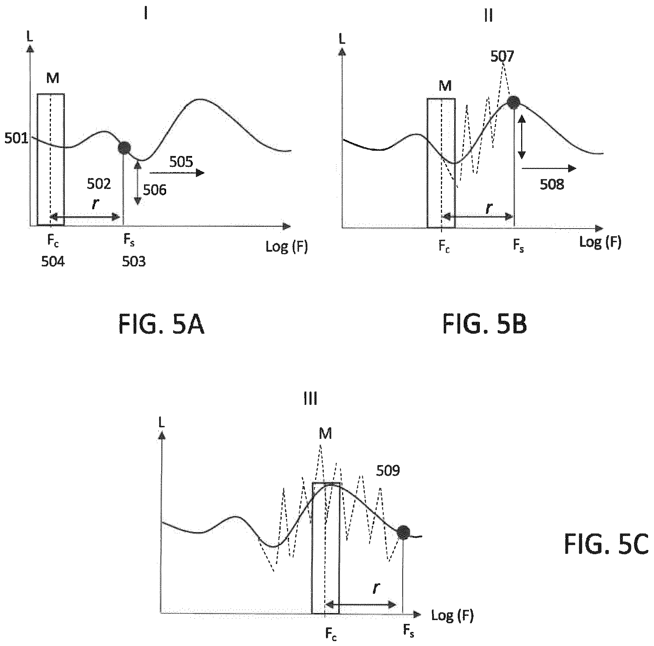

In some embodiments, other suprathreshold testing may be used. For example, a cross frequency masked threshold test is illustrated in FIGS. 5A-C. The y-axis represents the amplitude of the depicted signals, which include a noise masking probe M 504 and a tone signal probe 503. The x-axis is logarithmic in frequency F. As illustrated, noise masking probe M 504 has a center frequency $F_c$ and is kept at a fixed amplitude while being swept in frequency (i.e. the left to right progression seen in the graphs of FIGS. 5A-C). In some embodiments, the absolute width of the masking probe M 504 is dynamic, e.g. 0.2 octaves on either side of the center frequency $F_c$. Tone signal probe 503 has a frequency $F_s$ and a variable amplitude, i.e. an amplitude that is varied or adjusted while tone signal probe 503 is being swept in frequency, with an example variability or range of variability illustrated via arrow 506. In some embodiments, the rate of variation of amplitude of tone signal probe 503 is independent of the rate at which the masking probe 504 and tone signal probe 503 are frequency swept, although in other embodiments a relationship is contemplated, as will be explained in greater depth below. While performing frequency sweeping of the tone signal probe 503 and the masking probe 504, a fixed frequency ratio r is maintained, indicated in FIG. 5A at 502 and simply as 'r' elsewhere. In some embodiments, the fixed frequency ratio r is given by $[F_s/F_c]$ where $1.0 \leq r \leq 1.5$, although other ratio values may be utilized without departing from the scope of the present disclosure. As illustrated, masking probe 504 and signal probe 503 are then swept 505, 508 simultaneously to higher frequencies while Bekesy-style user responses 507, 509 are recorded and then interpolated to generate curve 501.

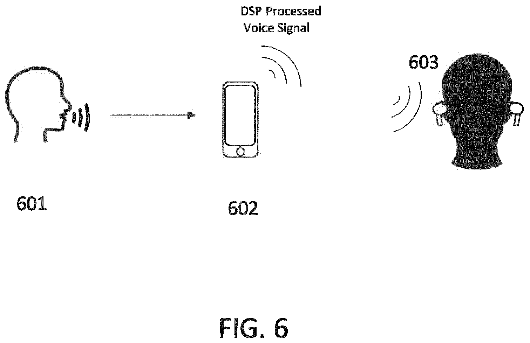

FIG. 6 illustrates an exemplary embodiment of a hearing assistive device according to one or more aspects of the present disclosure. As illustrated, a speaker's voice 601 is picked up by at least one microphone on mobile device 602 (although it is noted that the hearing assistive of the present disclosure is not limited to spoken voices and may be applied to various other sounds or noises recorded by the at least one microphone of mobile device 602 and/or otherwise received by the mobile device). After the voice audio signal is recorded by the at least one microphone, the signal is processed by a sound enhancement algorithm on mobile device 602 and the processed voice audio signal (shown here as a DSP Processed Voice Signal) is outputted to user's earphones 603, where the audio is outputted on the earphones' transducers.

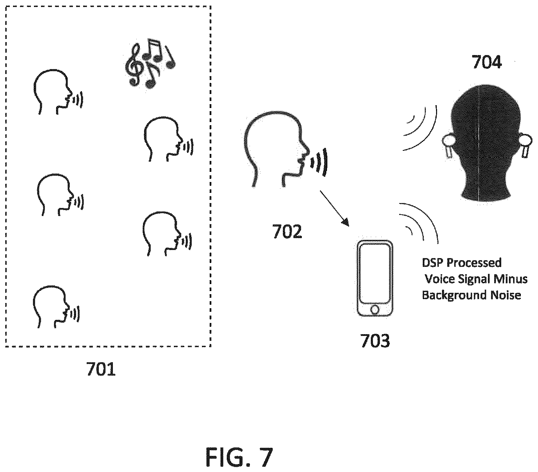

FIG. 7 illustrates another exemplary embodiment of an hearing assistive device according to aspects of the present disclosure. A speaker's voice 702 is picked up by at least one microphone on mobile device 703 against background noise 701, e.g., shown here as various other conversations or voices that are not that of the speaker of interest. Various processing techniques may be used to reduce background noise, such as directional processing (e.g., attenuating sound originating not originating directly in front of the user) and / or digital noise reduction (e.g., attenuating sounds that have the typical characteristics of noise regardless of the direction of arrival). After the voice audio signal is processed by the sound enhancement algorithm on mobile device 702, the voice audio signal is outputted to user's earphones 704, where the audio is outputted on the earphones' transducers.

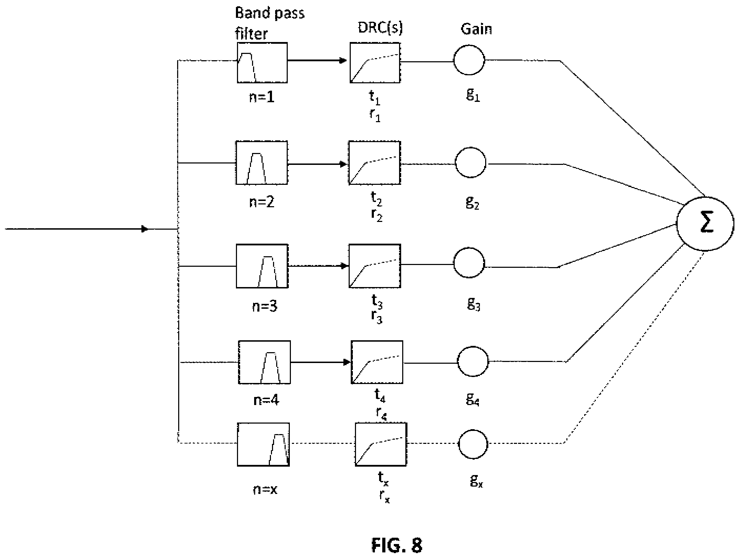

FIG. 8 illustrates an exemplary multiband dynamic range compressor (DRC), which may be used to personalize sound for an audio stream. Here, each subband (n = 1, ..., x) contains at least variables threshold ($t_n$) and ratio ($r_n$) for the subband's dynamic range compressor and gain ($g_n$). Other circuitries may be used (see for example, commonly owned US Patent No. 10,199,047).

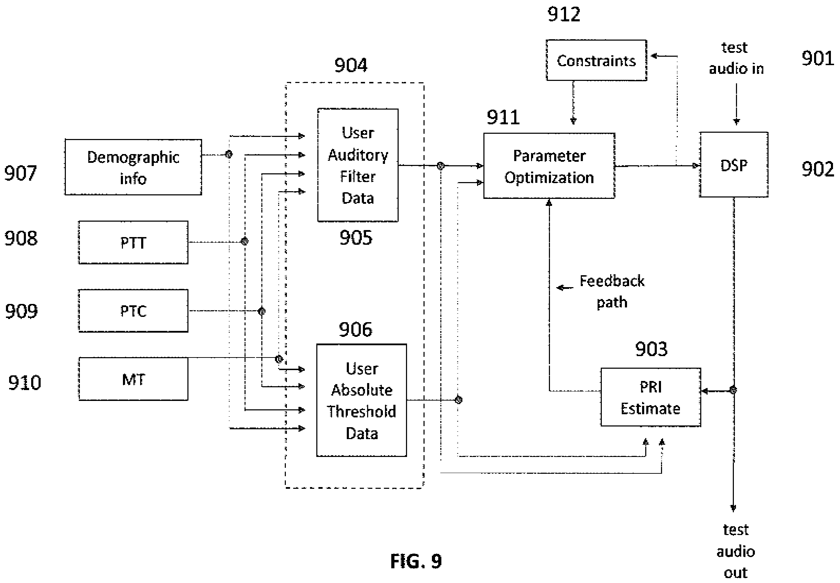

A PRI optimization approach may also be employed, details of which an example implementation are illustrated in FIG. 9. For example, DSP parameters in a multiband dynamic processor may be calculated by optimizing perceptually relevant information (e.g. perceptual entropy) through parameterization using user threshold and suprathreshold hearing data (see commonly owned US Patent No. US 10,455,335 and US Patent Application No. 16/538,541). Briefly, in order to optimally parameterize a multiband dynamic processor through perceptually relevant information, an audio sample 901, or body of audio samples representing a specific content type, is first processed by a parameterized multiband dynamics processor 902 (see also FIG. 8) and the perceptual entropy of the file is calculated 903 according to user threshold and suprathreshold hearing data 907. After calculation, the multiband dynamic processor is re-parameterized 911 according to a given set of parameter heuristics, derived from optimization, and from this - the audio sample(s) is reprocessed 902 and the PRI calculated 903. In other words, the multiband dynamics processor is configured to process the audio sample so that it has a higher PRI value for the particular listener, taking into account the individual listener's threshold and suprathreshold information 907. To this end, parameterization of the multiband dynamics processor is adapted to increase the PRI of the processed audio sample over the unprocessed audio sample. The parameters of the multiband dynamics processor are determined by an optimization process that uses PRI as its optimization criteria. Optionally, the PRI optimization process may be subject to constraints 912 to make the optimization process more efficient and worthwhile. This is performed by evaluating parameters within a given set of criteria to direct the end result to a level of signal manipulation that the end user deems tolerable (e.g. using EQ coloration criteria or against harmonic distortion and noise criteria to limit the optimization space, see commonly owned US Patent Application No. 16/538,541).

PRI can be calculated according to a variety of methods. One such method, also called perceptual entropy, generally comprises: transforming a sampled window of audio signal into the frequency domain, obtaining masking thresholds using psychoacoustic rules by performing critical band analysis, determining noise-like or tone-like regions of the audio signal, applying thresholding rules for the signal, and then accounting for absolute hearing thresholds. Following this, the number of bits required to quantize the spectrum without introducing perceptible quantization error is determined. For instance, Painter & Spanias disclose a formulation for perceptual entropy in units of bits/s, which is closely related to ISO/IEC MPEG-1 psychoacoustic model 2 [see e.g., Painter & Spanias, Perceptual Coding of Digital Audio, Proc. Of IEEE, Vol. 88, No. 4 (2000); see also generally Moving Picture Expert Group standards https://mpeg.chiariglione.org/standards; both documents included by reference].

Various optimization methods are possible to maximize the PRI of audio samples, depending on the type of the applied audio processing function such as the above-mentioned multiband dynamics processor. For example, a subband dynamic compressor may be parameterized by compression threshold, attack time, gain and compression ratio for each subband, and these parameters may be determined by the optimization process. In some cases, the effect of the multiband dynamics processor on the audio signal is nonlinear and an appropriate optimization technique such as gradient descend is required. The number of parameters that need to be determined may become large, e.g. if the audio signal is processed in many subbands and a plurality of parameters needs to be determined for each subband. In such cases, it may not be practicable to optimize all parameters simultaneously and a sequential approach for parameter optimization may be applied. Although sequential optimization procedures do not necessarily result in the optimum parameters, the obtained parameter values result in increased PRI over the unprocessed audio sample, thereby improving the listener's listening experience.

Other parameterization processes commonly known in the art may be used to calculate parameters based off user-generated threshold and suprathreshold information. For instance, common prescription techniques for linear and non-linear DSP may be employed. Well known procedures for linear hearing aid algorithms include POGO, NAL, and DSL. See, e.g., H. Dillon, Hearing Aids, 2nd Edition, Boomerang Press, 2012.

Fine tuning of any of the above-mentioned techniques may be estimated from manual fitting data. For instance, it is common in the art to fit a multiband dynamic processor according to series of subjective tests 704 given to a patient in which parameters are adjusted according to a patient's responses, e.g. a series of A/B tests, decision tree paradigms, 2D exploratory interface, in which the patient is asked which set of parameters subjectively sounds better. This testing ultimately guides the optimal parameterization of the DSP.

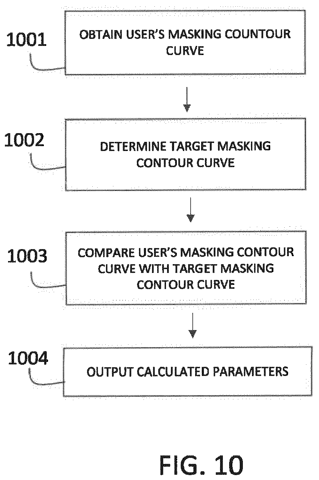

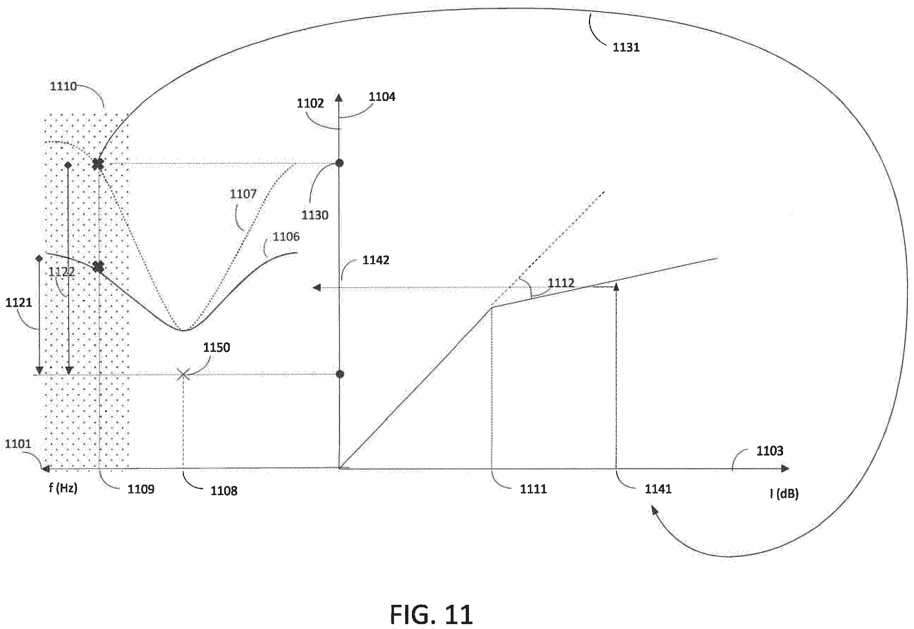

FIGS. 10 and 11 demonstrate one example approach to configuring the ratio and threshold parameters for a frequency band in a multi-band compression system (see, e.g., commonly owned applications EP18200368.1 and US 16/201,839, the contents of which are herein incorporated by reference) based upon a target curve / target age (see also FIG. 3). Briefly, a user's masking contour curve is received 1001, a target masking curve is determined 1002, and is subsequently compared with the user masking contour curve 1001 in order to determine and output user-calculated DSP parameter sets 1004.

FIG. 11 combines the visualization of a user masking contour curve 1106 for a listener (listener) and a target masking contour curve 1107 of a probe tone 1150 (with the x-axis 1101 being frequency, and the y-axis 1102 being the sound level in dB SPL or HL) with an input/output graph of a compressor showing the input level 1103 versus the output level 1104 of a sound signal, in decibels relative to full scale (dB FS). The bisecting line in the input/output graph represents a 1:1 (unprocessed) output of the input signal with gain 1.

The parameters of the multi-band compression system in a frequency band are threshold 1111 and gain 1112. These two parameters are determined from the user masking contour curve 1406 for the listener and target masking contour curve 1107. The threshold 1111 and ratio 1112 must satisfy the condition that the signal-to-noise ratio 1121 (SNR) of the user masking contour curve 1106 at a given frequency 1109 is greater than the SNR 1122 of the target masking contour curve 1107 at the same given frequency 1109. Note that the SNR is herein defined as the level of the signal tone compared to the level of the masker noise. The broader the curve will be, the greater the SNR. The given frequency 1109 at which the SNRs 1121 and 1122 are calculated may be arbitrarily chosen, for example, to be beyond a minimum distance from the probe tone frequency 1408.

The sound level 1130 (in dB) of the target masking contour curve 1107 at a given frequency corresponds (see bent arrow 1131) to an input sound level 1141 entering the compression system. The objective is that the sound level 1142 outputted by the compression system will match the user masking contour curve 1106, i.e., that this sound level 1142 is substantially equal to the sound level (in dB) of the user masking contour curve 1106 at the given frequency 1109. This condition allows the derivation of the threshold 1111 (which has to be below the input sound level 1141) and the ratio 1112. In other words, input sound level 1141 and output sound level 1142 determine a reference point of the compression curve. As noted above, threshold 1111 must be selected to be lower than input sound level 1141 - if it is not, there will be no change, as below the threshold of the compressor, the system is linear). Once the threshold 1111 is selected, the ratio 1112 can be determined from the threshold and the reference point of the compression curve.

In the context of the present disclosure, a masking contour curve is obtained from a user hearing test. A target masking contour curve 1107 is interpolated from at least the user masking contour curve 1106 and a reference masking contour curve, representing the curve of a normal hearing individual. In some embodiments, the target masking contour curve 1107 is preferred over a reference curve because fitting an audio signal to a reference curve is not necessarily optimal. Depending on the initial hearing ability of the listener, fitting the processing according to a reference curve may cause an excess of processing to spoil the quality of the signal. The objective is to process the signal in order to obtain a good balance between an objective benefit and a good sound quality.

The given frequency 1109 is then chosen. It may be chosen arbitrarily, e.g., at a certain distance from the tone frequency 1108 The corresponding sound levels of the listener and target masking contour curves are determined at this given frequency 1109. The value of these sound levels may be determined graphically on the y-axis 1102.

The right panel in FIG. 11 (see the contiguous graph) illustrates a hard knee DRC, with a threshold 1111 and a ratio 1112 as parameters that need to be determined. An input sound signal having a sound level 1130/1141 at a given frequency 1109 enters the compression system (see bent arrow 1131 indicating correspondence between 1130/1141). The sound signal should be processed by the DRC in such a way that the outputted sound level is the sound level of the user masking contour curve 1106 at the given frequency 1109. The threshold 1111 should not exceed the input sound level 1141, otherwise compression will not occur. Multiple sets of threshold and ratio parameters are possible. Preferred sets can be selected depending on a fitting algorithm and/or objective fitting data that have proven to show the most benefit in terms of sound quality. For example, either one of the threshold 1111 and ratio 1112 may be chosen to have a default value, and the respective other one of the parameters can then be determined by imposing the above-described condition.

In some embodiments, content-specific DSP parameter sets may be calculated indirectly from a user hearing test based on preexisting entries or anchor points in a server database. An anchor point comprises a typical hearing profile constructed based at least in part on demographic information, such as age and sex, in which DSP parameter sets are calculated and stored on the server to serve as reference markers. Indirect calculation of DSP parameter sets bypasses direct parameter sets calculation by finding the closest matching hearing profile(s) and importing (or interpolating) those values for the user.

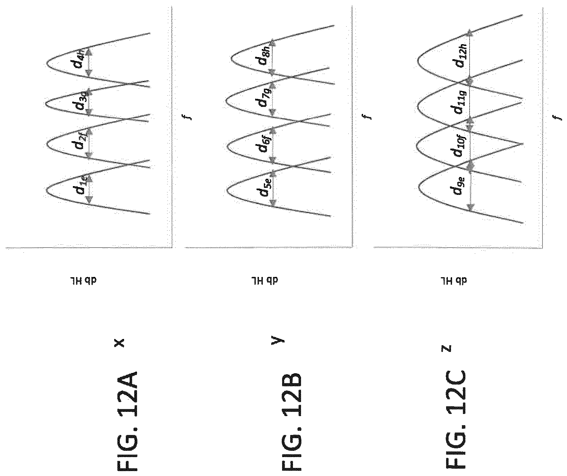

FIGS. 12A-C illustrate three conceptual user masked threshold (MT) curves for users x, y, and z, respectively. The MT curves are centered at frequencies a-d, each with curve width d, which may be used to as a metric to measure the similarity between user hearing data. For instance, a root mean square difference calculation may be used to determine if user y's hearing data is more similar to user x's or user z's, e.g. by calculating:

$$(\sqrt{(d5a - d1a)^2 + (d6b - d2b)^2 \dots} < \sqrt{(d5a - d9a)^2 + (d6b - d10b)^2 \dots}$$

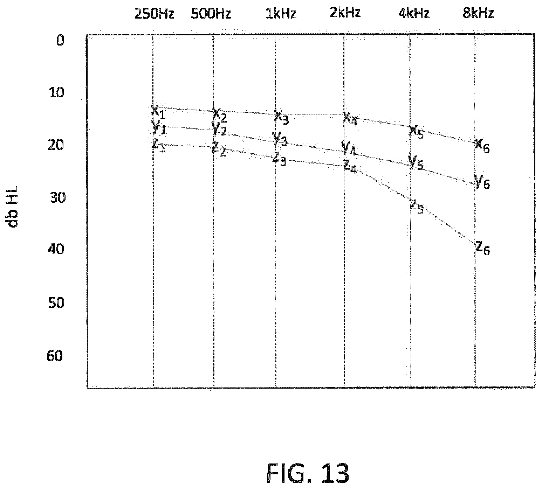

FIG. 13 illustrates three conceptual audiograms of users x, y and z, each with pure tone threshold values 1-5. Similar to above, a root mean square difference measurement may also be used to determine, for example, if user y's hearing data is more similar to user x's than user z's, e.g., by calculating:

$$\left(\sqrt{(y1 - x1)^2 + (y2 - x2)^2 \dots} \quad < \sqrt{(y1 - z1)^2 + (y2 - z2)^2 \dots}\right)$$

As would be appreciated by one of ordinary skill in the art, other methods may be used to quantify similarity amongst user hearing profile graphs, where the other methods can include, but are not limited to, methods such as a Euclidean distance measurements, e.g. $((y1 - x1) + (y2 - x2) \dots > (y1 - x1) + (y2 - x2)) \dots$ or other statistical methods known in the art. For indirect DSP parameter set calculation, then, the closest matching hearing profile(s) between a user and other preexisting database entries or anchor points can then be used.

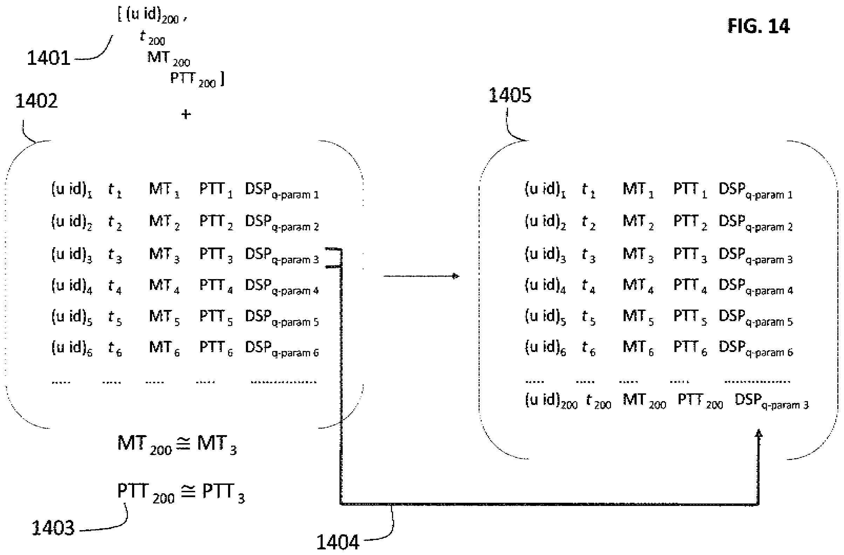

FIG. 14 illustrates an exemplary embodiment for calculating sound enhancement parameter sets for a given algorithm based on preexisting entries and/or anchor points. Here, server database entries 1402 are surveyed to find the best fit(s) with user hearing data input 1401, represented as $MT_{200}$ and $PTT_{200}$ for $(u\_id)_{200}$. This may be performed by the statistical techniques illustrated in FIGS. 12 and 13. In the example of FIG. 14, $(u\_id)_{200}$ hearing data best matches $MT_3$ and $PTT_3$ data 1403. To this extent, $(u\_id)_3$ associated parameter sets, $[DSP_{q\text{-}param\ 3}]$, are then used for the $(u\_id)_{200}$ parameter set entry, illustrated here as $[(u\_id)_{200}, t_{200}, MT_{200}, PTT_{200}, DSP_{q\text{-}param\ 3}]$.

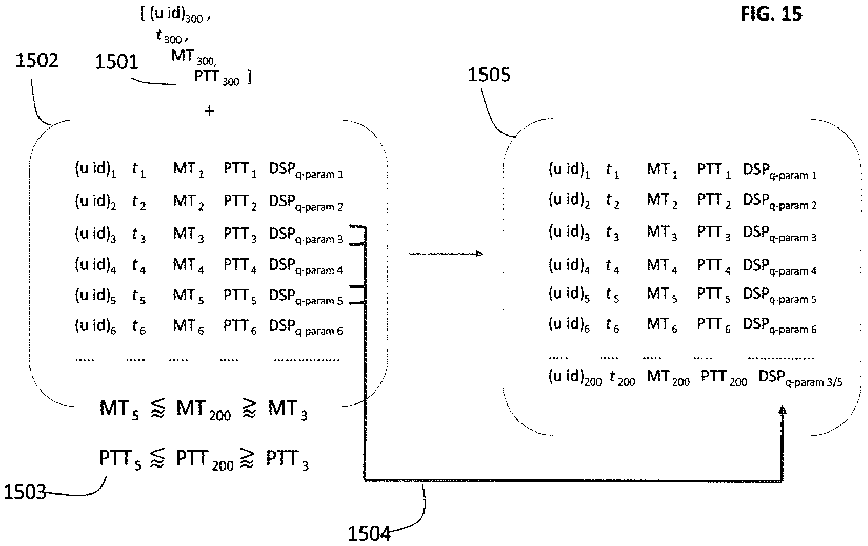

FIG. 15 illustrates an exemplary embodiment for calculating sound enhancement parameter sets for a given algorithm based on preexisting entries or anchor points, according to aspects of the present disclosure. Here, server database entries 1502 are employed to interpolate 1504 between two nearest fits 1500 with user hearing data input 1501 $MT_{300}$ and $PT_{300}$ for $(u\_id)_{300}$. In this example, the $(u\_id)_{300}$ hearing data fits nearest between: $MT_5 \lesseqgtr MT_{200} \gtreqless$ $MT_3$ and $PTT_5 \lesseqgtr PTT_{200} \gtreqless PTT_3$ 1503. To this extent, $(u\_id)_3$ and $(u\_id)_5$ parameter sets are interpolated to generate a new set of parameters for the $(u\_id)_{300}$ parameter set entry, represented here as $[(u\_id)_{200}, t_{200}, MT_{200}, PTT_{200}, DSP_{q\text{-}param3/5}]$ 1505. In a further embodiment, interpolation may be performed across multiple data entries to calculate sound enhancement parameters.

DSP parameter sets may be interpolated linearly, e.g., a DRC ratio value of 0.7 for user 5 $(u\_id)_5$ and 0.8 for user 3 $(u\_id)_3$ would be interpolated as 0.75 for user 200 $(u\_id)_{200}$ in the example of FIG. 14 (and/or a user in the context of FIGS. 12A-C), assuming user 200's hearing data was halfway in-between that of users 3 and 5. In some embodiments, DSP parameter sets may also be interpolated non-linearly, for instance using a squared-function, e.g. a DRC ratio value of 0.6 for user 5 and 0.8 for user 3 would be non-linearly interpolated as 0.75 for user 200 in the example of FIG. 14 (and/or a user in the context of FIGS. 12A-C).

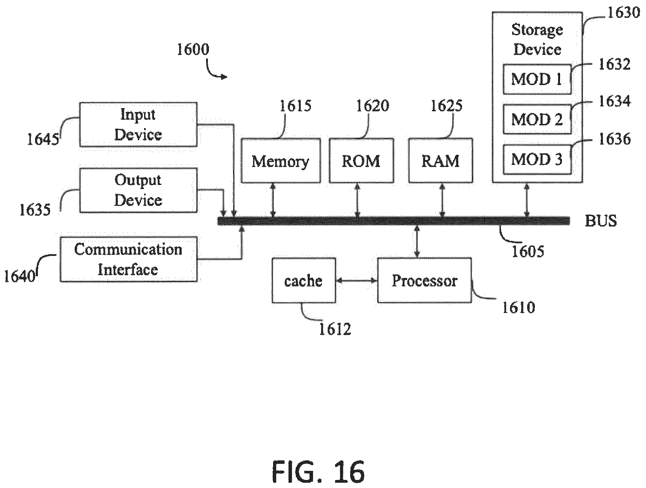

FIG. 16 shows an example of computing system 1600, which can be for example any computing device making up (e.g., mobile device 100, server, etc.) or any component thereof in which the components of the system are in communication with each other using connection 1605. Connection 1605 can be a physical connection via a bus, or a direct connection into processor 1610, such as in a chipset architecture. Connection 1605 can also be a virtual connection, networked connection, or logical connection.

In some embodiments computing system 1600 is a distributed system in which the functions described in this disclosure can be distributed within a datacenter, multiple datacenters, a peer network, etc. In some embodiments, one or more of the described system components represents many such components each performing some or all of the function for which the component is described. In some embodiments, the components can be physical or virtual devices.

Example system 1600 includes at least one processing unit (CPU or processor) 1610 and connection 1605 that couples various system components including system memory 1615, such as read only memory (ROM) 1620 and random access memory (RAM) 1625 to processor 1610. Computing system 1600 can include a cache of high-speed memory 1612 connected directly with, in close proximity to, or integrated as part of processor 1610.

Processor 1610 can include any general-purpose processor and a hardware service or software service, such as services 1632, 1634, and 1636 stored in storage device 1630, configured to control processor 1610 as well as a special-purpose processor where software instructions are incorporated into the actual processor design. Processor 1610 may essentially be a completely self-contained computing system, containing multiple cores or processors, a bus, memory controller, cache, etc. A multi-core processor may be symmetric or asymmetric.

To enable user interaction, computing system 1600 includes an input device 1645, which can represent any number of input mechanisms, such as a microphone for speech, a touch-sensitive screen for gesture or graphical input, keyboard, mouse, motion input, speech, etc. Computing system 1600 can also include output device 1635, which can be one or more of a number of output mechanisms known to those of skill in the art. In some instances, multimodal systems can enable a user to provide multiple types of input/output to communicate with computing system 1600.

Computing system 1600 can include communications interface 1640, which can generally govern and manage the user input and system output. There is no restriction on operating on any particular hardware arrangement and therefore the basic features here may easily be substituted for improved hardware or firmware arrangements as they are developed.

Storage device 1630 can be a non-volatile memory device and can be a hard disk or other types of computer readable media which can store data that are accessible by a computer, flash memory cards, solid state memory devices, digital versatile disks, cartridges, random access memories (RAMs), read only memory (ROM), and/or some combination of these devices.

The storage device 1630 can include software services, servers, services, etc., that when the code that defines such software is executed by the processor 1610, it causes the system to perform a function. In some embodiments, a hardware service that performs a particular function can include the software component stored in a computer-readable medium in connection with the necessary hardware components, such as processor 1610, connection 1605, output device 1635, etc., to carry out the function.

It should be further noted that the description and drawings merely illustrate the principles of the proposed device. Those skilled in the art will be able to implement various arrangements that, although not explicitly described or shown herein, embody the principles of the invention and are included within its spirit and scope. Furthermore, all examples and embodiment outlined in the present document are principally intended expressly to be only for explanatory purposes to help the reader in understanding the principles of the proposed device. Furthermore, all statements herein providing principles, aspects, and embodiments of the invention, as well as specific examples thereof, are intended to encompass equivalents thereof.

Methods according to the above-described examples can be implemented using computer-executable instructions that are stored or otherwise available from computer readable media. Such instructions can comprise, for example, instructions and data which cause or otherwise configure a general-purpose computer, special purpose computer, or special purpose processing device to perform a certain function or group of functions. Portions of computer resources used can be accessible over a network. The computer executable instructions may be, for example, binaries, intermediate format instructions such as assembly language, firmware, or source code. Examples of computer-readable media that may be used to store instructions, information used, and/or information created during methods according to described examples include magnetic or optical disks, flash memory, USB devices provided with non-volatile memory, networked storage devices, and so on.

Devices implementing methods according to these disclosures can comprise hardware, firmware and/or software, and can take any of a variety of form factors. Typical examples of such form factors include laptops, smart phones, small form factor personal computers, personal digital assistants, rackmount devices, standalone devices, and so on. Functionality described herein also can be embodied in peripherals or add-in cards. Such functionality can also be implemented on a circuit board among different chips or different processes executing in a single device, by way of further example. The instructions, media for conveying such instructions, computing resources for executing them, and other structures for supporting such computing resources are means for providing the functions described in these disclosures.

Although a variety of examples and other information was used to explain aspects within the scope of the appended claims, no limitation of the claims should be implied based on particular features or arrangements in such examples, as one of ordinary skill would be able to use these examples to derive a wide variety of implementations. Further and although some subject matter may have been described in language specific to examples of structural features and/or method steps, it is to be understood that the subject matter defined in the appended claims is not necessarily limited to these described features or acts. For example, such functionality can be distributed differently or performed in components other than those identified herein. Rather, the described features and steps are disclosed as examples of components of systems and methods within the scope of the appended claims.

## Claims

1. A method for ambient sound enhancement on a mobile device, the method comprising:

   generating a user hearing profile;
   calculating at least one set of ambient sound enhancement digital signal processing (DSP) parameters for a sound enhancement algorithm, the calculation of the ambient sound enhancement DSP parameters based at least in part on the user hearing profile;
   in response to a user initiating an ambient sound enhancement function on a mobile computing device, retrieving the at least one set of calculated ambient sound enhancement DSP parameters;
   capturing ambient sound with at least one microphone of the mobile computing device;
   processing the captured ambient sound with an ambient sound enhancement DSP to generate a DSP enhanced processed audio signal, wherein the ambient sound enhancement DSP is parameterized with the retrieved set of calculated ambient sound enhancement DSP parameters; and

outputting the DSP enhanced processed audio signal to a transducer of the mobile device.

2. The method of claim 1, wherein capturing the ambient sound is performed in substantially real time with one or more of:

processing the captured ambient sound with the ambient sound enhancement DSP to generate the DSP enhanced processed audio signal; and

outputting the DSP enhanced processed audio signal to the transducer of the mobile device.

3. The method of claim 1 or 2, wherein the retrieved set of calculated ambient sound enhancement DSP parameters corresponds to a sound enhancement algorithm associated with the user's mobile computing device or indicated by a user input to the mobile computing device.

4. The method of claim 3, wherein the sound enhancement algorithm associated with the user's mobile computing device is selected from a plurality of available sound enhancement algorithms configured in a local storage of the user's mobile computing device.

5. The method of claim 4, wherein the selection of the sound enhancement algorithm from the plurality of sound enhancement algorithms is based at least in part on an analysis of the ambient sound captured by the user's mobile computing device.

6. The method of any previous claim, further comprising one or more of processing the captured ambient sound to:

attenuate sound not originating in front of the user or the microphone of the user's mobile computing device that captured the ambient sound, by applying a directional processing algorithm to the captured ambient sound or the DSP enhanced processed audio signal; and

attenuate sounds that have typical characteristics of noise, regardless of the direction of arrival, by applying one or more digital noise reduction algorithms.

7. The method of any previous claim, wherein the user hearing profile is generated by conducted at least one hearing test on a mobile computing device.

8. The method of claim 7, wherein the mobile computing device is the mobile computing device associated with the user.

9. The method of claim 7, wherein the hearing test is one or more of a masked threshold test (MT test), a pure tone threshold test (PTT test), a psychophysical tuning curve test (PTC test), or a cross frequency simultaneous masking test (xF-SM test).

10. The method of any previous claim, wherein the user hearing profile is generated at least in part by analyzing a user input of demographic information to thereby interpolate a representative hearing profile.

11. The method of claim 10, wherein the user input of demographic information includes an age of the user.

12. The method of any previous claim, wherein:

the sound enhancement algorithm is a multiband dynamic processor; and

the at least one set of calculated ambient sound enhancement DSP parameters includes one or more ratio values and gain values.

13. The method of any previous claim, wherein:

the at least one set of calculated ambient sound enhancement DSP parameters is stored on a remote server; and

retrieving the at least one set of calculated ambient sound enhancement DSP parameters comprises receiving a requested set of calculated ambient sound enhancement DSP parameters at the mobile computing device from the remote server.

14. The method of any previous claim, wherein:

the at least one set of calculated ambient sound enhancement DSP parameters is stored locally on the mobile computing device; and
retrieving the at least one set of calculated ambient sound enhancement DSP parameters comprises accessing a local storage of the mobile computing device.

15. The method of any previous claim, wherein calculating the at least one set of ambient sound enhancement DSP parameters is performed on a remote server.

16. The method of any previous claim, wherein calculating the at least one set of ambient sound enhancement DSP parameters is performed by a processor of the user's mobile computing device.

17. The method of any previous claim, wherein the hearing test measures masking threshold curves within a range of frequencies from 250 Hz to 12 kHz.

18. The method of any previous claim, wherein the at least one set of calculated ambient sound enhancement DSP parameters is determined via one or more of:

a best fit of the user hearing profile with previously inputted hearing data within a database; or a fitted mathematical function derived from plotted hearing and DSP parameter data.

19. The method of claim 18, wherein the parameters associated with the best fit of the user hearing profile and the previously inputted hearing data are selected to correspond to a user's parameters, and/or wherein the best fit is determined by one or more of average Euclidean distance and root mean square difference.

20. A device for ambient sound enhancement on a mobile device, comprising a processor and a memory storing instructions for execution on the processor, the instructions for configuring the processor to perform the method of any of the previous method claims.

FIG. 1

Average MT curves across absolute threshold levels at 4kHz

+60dB (~85y)

+50dB (~80y)

+40dB (~75y)

+30dB (~68y)

+20dB (~60y)

+10dB (~50y)

+0dB (~25y)

MT level [dB re $L_{masker}$]

Frequency [re $F_{masker}$]

FIG. 2

# FIG. 3

313

301 MT
302 xF-SM
303 PTC
304 PTT
305 OTHER

306 CONDUCT HEARING TEST ON MOBILE DEVICE AND / OR INPUT HEARING AGE

307 CALCULATE AT LEAST ONE SET OF DSP PARAMETERS FOR AT LEAST ONE SOUND ENHANCEMENT ALGORITHM

308 STORE DSP PARAMETERS ON DEVICE OR SERVER DATABASE

309 ASSISTED HEARING INITIATED BY USER

310 RETRIEVE DSP PARAMETERS

311 OUTPUT PARAMETERS TO AUDIO OUTPUT DEVICE'S SOUND EHANCEMENT DSP

312 AMBIENT NOISE CAPTURED FROM AT LEAST ONE MICROPHONE ON A MOBILE DEVICE

314 PROCESS AMBIENT SOUND WITH PARAMETERIZED SOUND ENHANCEMENT DSP FOR ASSISTED HEARING

FIG. 4A

FIG. 4B

FIG. 5A

FIG. 5B

FIG. 5C

EP 3 896 999 A1

DSP Processed
Voice Signal

603

601            602

FIG. 6

**FIG. 7**

FIG. 8

FIG. 9

1001 — OBTAIN USER'S MASKING COUNTOUR CURVE

1002 — DETERMINE TARGET MASKING CONTOUR CURVE

1003 — COMPARE USER'S MASKING CONTOUR CURVE WITH TARGET MASKING CONTOUR CURVE

1004 — OUTPUT CALCULATED PARAMETERS

FIG. 10

FIG. 11

FIG. 12A

FIG. 12B

FIG. 12C

FIG. 13

EP 3 896 999 A1

FIG. 14

1401 —

$[\ (u\ id)_{200},$
$t_{200}$
$MT_{200}$
$PTT_{200}\ ]$

$+$

1402

| | | | | |
|---|---|---|---|---|
| $(u\ id)_1$ | $t_1$ | $MT_1$ | $PTT_1$ | $DSP_{q\text{-}param\ 1}$ |
| $(u\ id)_2$ | $t_2$ | $MT_2$ | $PTT_2$ | $DSP_{q\text{-}param\ 2}$ |
| $(u\ id)_3$ | $t_3$ | $MT_3$ | $PTT_3$ | $DSP_{q\text{-}param\ 3}$ |
| $(u\ id)_4$ | $t_4$ | $MT_4$ | $PTT_4$ | $DSP_{q\text{-}param\ 4}$ |
| $(u\ id)_5$ | $t_5$ | $MT_5$ | $PTT_5$ | $DSP_{q\text{-}param\ 5}$ |
| $(u\ id)_6$ | $t_6$ | $MT_6$ | $PTT_6$ | $DSP_{q\text{-}param\ 6}$ |
| ..... | ..... | ..... | ..... | ................. |

$MT_{200} \cong MT_3$

$PTT_{200} \cong PTT_3$

1403 —

1405

| | | | | |
|---|---|---|---|---|
| $(u\ id)_1$ | $t_1$ | $MT_1$ | $PTT_1$ | $DSP_{q\text{-}param\ 1}$ |
| $(u\ id)_2$ | $t_2$ | $MT_2$ | $PTT_2$ | $DSP_{q\text{-}param\ 2}$ |
| $(u\ id)_3$ | $t_3$ | $MT_3$ | $PTT_3$ | $DSP_{q\text{-}param\ 3}$ |
| $(u\ id)_4$ | $t_4$ | $MT_4$ | $PTT_4$ | $DSP_{q\text{-}param\ 4}$ |
| $(u\ id)_5$ | $t_5$ | $MT_5$ | $PTT_5$ | $DSP_{q\text{-}param\ 5}$ |
| $(u\ id)_6$ | $t_6$ | $MT_6$ | $PTT_6$ | $DSP_{q\text{-}param\ 6}$ |
| ..... | ..... | ..... | ..... | ................. |
| $(u\ id)_{200}$ | $t_{200}$ | $MT_{200}$ | $PTT_{200}$ | $DSP_{q\text{-}param\ 3}$ |

1404 —

EP 3 896 999 A1

26

**FIG. 15**

$[ (u\,id)_{300},$

$t_{300},$

$MT_{300},$

$PTT_{300} ]$

$+$

1502    1501                                            1505

| | | | | |
|---|---|---|---|---|
| $(u\,id)_1$ | $t_1$ | $MT_1$ | $PTT_1$ | $DSP_{q\text{-}param\,1}$ |
| $(u\,id)_2$ | $t_2$ | $MT_2$ | $PTT_2$ | $DSP_{q\text{-}param\,2}$ |
| $(u\,id)_3$ | $t_3$ | $MT_3$ | $PTT_3$ | $DSP_{q\text{-}param\,3}$ |
| $(u\,id)_4$ | $t_4$ | $MT_4$ | $PTT_4$ | $DSP_{q\text{-}param\,4}$ |
| $(u\,id)_5$ | $t_5$ | $MT_5$ | $PTT_5$ | $DSP_{q\text{-}param\,5}$ |
| $(u\,id)_6$ | $t_6$ | $MT_6$ | $PTT_6$ | $DSP_{q\text{-}param\,6}$ |
| ...... | ...... | ...... | ...... | .................. |

| | | | | |
|---|---|---|---|---|
| $(u\,id)_1$ | $t_1$ | $MT_1$ | $PTT_1$ | $DSP_{q\text{-}param\,1}$ |
| $(u\,id)_2$ | $t_2$ | $MT_2$ | $PTT_2$ | $DSP_{q\text{-}param\,2}$ |
| $(u\,id)_3$ | $t_3$ | $MT_3$ | $PTT_3$ | $DSP_{q\text{-}param\,3}$ |
| $(u\,id)_4$ | $t_4$ | $MT_4$ | $PTT_4$ | $DSP_{q\text{-}param\,4}$ |
| $(u\,id)_5$ | $t_5$ | $MT_5$ | $PTT_5$ | $DSP_{q\text{-}param\,5}$ |
| $(u\,id)_6$ | $t_6$ | $MT_6$ | $PTT_6$ | $DSP_{q\text{-}param\,6}$ |
| ...... | ...... | ...... | ...... | .................. |
| $(u\,id)_{200}$ | $t_{200}$ | $MT_{200}$ | $PTT_{200}$ | $DSP_{q\text{-}param\,3/5}$ |

$MT_5 \lesseqgtr MT_{200} \gtreqless MT_3$

$PTT_5 \lesseqgtr PTT_{200} \gtreqless PTT_3$

1503

1504

FIG. 16

Europäisches
Patentamt

European
Patent Office

Office européen
des brevets

# EUROPEAN SEARCH REPORT

**Application Number**

EP 20 19 7642

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/241256 A1 (GOLDSTEIN STEVEN [US] ET AL) 23 September 2010 (2010-09-23)<br>* figures 3A, 5 *<br>* paragraphs [0002], [0019], [0092], [0105], [0111], [0113] - [0120], [0130], [0139] *<br>* paragraphs [0167], [0179] - [0184], [0181], [0188], [0286], [0291] * | 1-20 | INV.<br>H04R25/00<br>G10L21/0364 |
| X | US 2003/064746 A1 (RADER R SCOTT [US] ET AL) 3 April 2003 (2003-04-03)<br>* figures 1, 2, 8-10 *<br>* paragraphs [0039], [0040], [0052], [0053], [0059], [0212], [0216] * | 1-20 | |
| A | US 2020/029159 A1 (CLARK NICHOLAS R [GB]) 23 January 2020 (2020-01-23)<br>* paragraphs [0009], [0017], [0021], [0028], [0031], [0032], [0070], [0096] * | 7-12,16, 17 | |
| | | | **TECHNICAL FIELDS SEARCHED (IPC)**<br><br>H04R<br>A61B<br>G10L<br>G10H |

The present search report has been drawn up for all claims

| Place of search | Date of completion of the search | Examiner |
|---|---|---|
| The Hague | 16 November 2020 | Mendoza Lopez, Jorge |

EPO FORM 1503 03.82 (P04C01)

## ANNEX TO THE EUROPEAN SEARCH REPORT
## ON EUROPEAN PATENT APPLICATION NO.

EP 20 19 7642

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2020

| Patent document cited in search report | | Publication date | Patent family member(s) | | Publication date |
|---|---|---|---|---|---|
| US 2010241256 | A1 | 23-09-2010 | US 2010241256 A1 | | 23-09-2010 |
| | | | WO 2007137232 A2 | | 29-11-2007 |
| US 2003064746 | A1 | 03-04-2003 | CN 1589588 A | | 02-03-2005 |
| | | | EP 1438874 A1 | | 21-07-2004 |
| | | | JP 2005504470 A | | 10-02-2005 |
| | | | KR 20040053134 A | | 23-06-2004 |
| | | | US 2003064746 A1 | | 03-04-2003 |
| | | | US 2005260978 A1 | | 24-11-2005 |
| | | | US 2005260985 A1 | | 24-11-2005 |
| | | | WO 03026349 A1 | | 27-03-2003 |
| US 2020029159 | A1 | 23-01-2020 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 16851048 B
- US 10199047 B
- US 10455335 B
- US 538541
- EP 18200368
- US 16201839 B

### Non-patent literature cited in the description

- **PAINTER ; SPANIAS.** Perceptual Coding of Digital Audio. *Proc. Of IEEE,* 2000, vol. 88 (4
- **H. DILLON.** Hearing Aids. Boomerang Press, 2012